(19) Europäisches Patentamt
European Patent Office
Office européen des brevets

(11) EP 0 728 474 B1

# (12) FASCICULE DE BREVET EUROPEEN

(45) Date de publication et mention de la délivrance du brevet:
**19.11.1997 Bulletin 1997/47**

(51) Int. Cl.$^6$: **A61K 7/48**

(21) Numéro de dépôt: **96400238.0**

(22) Date de dépôt: **05.02.1996**

## (54) Utilisation des acides sulfoniques comme actifs anti-acneiques dans une composition cosmétique ou dermatologique

Verwendung von Sulfonsäuren als Anti-Aknemittel in kosmetischen oder dermatologischen Zusammensetzungen

Use of sulphonic acids as anti-acne agents in cosmetic or dermatological compositions

(84) Etats contractants désignés:
**DE ES FR GB IT**

(30) Priorité: **24.02.1995 FR 9502198**

(43) Date de publication de la demande:
**28.08.1996 Bulletin 1996/35**

(73) Titulaire: **L'OREAL**
**75008 Paris (FR)**

(72) Inventeur: **Fanchon, Chantal**
**F-75015 Paris (FR)**

(74) Mandataire: **Lhoste, Catherine**
**L'OREAL,**
**D.P.I.,**
**90 rue du Général Roguet**
**92583 Clichy Cédex (FR)**

(56) Documents cités:
**GB-A- 2 001 649**
**GB-A- 2 150 436**
**GB-A- 2 200 552**

EP 0 728 474 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.14.23/3.4

## Description

La présente invention a pour objet l'utilisation d'acides sulfoniques comme actifs anti-acnéiques dans des compositions à application topique.

L'acné est l'une des maladies affectant le plus souvent, et à des degrés divers, la population juvénile entre 15 et 30 ans. L'acné est essentiellement la conséquence de deux phénomènes intriqués : la formation du comédon et l'inflammation péricomédonienne ou folliculite, la formation du comédon étant elle-même la conséquence de deux phénomènes : l'obstruction du canal pilosébacé et la production accrue de sébum par les glandes sébacées, donnant lieu à la formation de points noirs. L'acné n'est pas inflammatoire au stade de la formation du comédon, mais le devient par suite de la prolifération de germes résultant de la rétention séborrhéique et de l'hyperproduction de sébum. Les germes en cause sont notamment des germes anaérobies diphthéroïdes tels que les *Propionibacteria (acnés, granulosum, avidum).* Au stade de la formation du comédon, le traitement peut consister simplement à épurer la peau, tandis qu'au stade d'inflammation, il convient d'effectuer un traitement anti-inflammatoire.

Pour traiter l'acné, on utilise généralement des agents kératolytiques tels que les rétinoïdes, et notamment l'acide rétinoïque, des anti-inflammatoires tels que les peroxydes, et notamment le peroxyde de benzoyle, et des agents antiséborrhéiques.

Ces actifs présentent l'inconvénient d'être assez irritants, et cet effet irritant est d'autant plus ressenti par le sujet traité que celui-ci a la peau sensible.

On constate donc que subsiste le besoin d'actifs anti-acnéiques ayant une action au moins aussi efficace que les composés de l'art antérieur, mais ne présentant pas leurs inconvénients.

C'est ce qu'apporte la présente invention.

La demanderesse a découvert que pouvaient être utilisés dans une composition topique en tant qu'actifs anti-acnéiques les composés présentant au moins une fonction acide sulfonique partiellement non neutralisée, c'est-à-dire non complètement neutralisée.

Ces composés se sont révélés être des agents kératolytiques efficaces pour le traitement de l'acné aussi bien pour empêcher la formation des comédons que pour traiter l'acné au stade inflammatoire.

Certains de ces acides sulfoniques ont été précédemment utilisés dans des compositions antisolaires pour protéger la peau du rayonnement ultraviolet, c'est-à-dire pour protéger la peau des érythèmes et des brûlures provoqués par les radiations lumineuses de longueur d'onde comprise entre 280 et 400 nm, plus particulièrement entre 280 et 360 nm. De telles compositions sont notamment décrites dans les documents suivants : US 4.585.597, FR 2.236.515, 2.282.426, 2.645.148, 2.430.938 et 2.592.380.

Ces acides sulfoniques sont utilisés généralement sous leur forme totalement neutralisée, forme inapte pour la lutte contre l'acné.

Par ailleurs, le document GB-A-2001649 décrit l'utilisation du tris-(N-oxypyridine-2-thiolate) d'aluminium, auquel on peut ajouter comme additif du tri-(campho-10-sulfonate) d'aluminium, pour traiter les manifestations pathologiques où un microorganisme est impliqué.

La présente invention a donc pour objet l'utilisation d'au moins un composé présentant au moins une fonction acide sulfonique non complètement neutralisée comme actif anti-acnéique dans ou pour la fabrication d'une composition topique.

La présente invention a encore pour objet l'utilisation d'au moins un composé présentant au moins une fonction acide sulfonique non complètement neutralisée, dans ou pour la fabrication d'une composition topique pour traiter la rétention séborrhéique et l'hyperproduction de sébum de la peau.

La présente invention a aussi pour objet un procédé de traitement cosmétique de l'acné consistant à appliquer sur la peau un composé présentant au moins une fonction acide sulfonique non complètement neutralisée.

La présente invention a encore pour objet un procédé de traitement thérapeutique de l'acné consistant à appliquer sur la peau un composé présentant au moins une fonction acide sulfonique non complètement neutralisée.

Les composés selon l'invention doivent présenter une fonction acide sulfonique non complètement neutralisée, ce qui signifie que le taux de neutralisation de ce composé acide dans la composition ne doit pas être total et que si la composition contient un neutralisant, celui-ci doit être en une quantité insuffisante pour neutraliser toutes les fonctions acides sulfoniques présentes dans la composition. Plus le taux de neutralisation est faible, plus l'action kératolytique proprement dite de la composition est importante.

En pratique, les compositions selon l'invention ont un pH inférieur à 6, de préférence allant de 1 à 5, et encore mieux de 2 à 4.

La présente invention présente un intérêt tout particulier lorsque l'on met en oeuvre les acides sulfoniques qui sont utilisés comme filtre solaire dans l'art antérieur sous leur forme neutralisée. En effet, dans ce cas là, la peau est également protégée des effets nocifs du rayonnement ultraviolet, ceci grâce à la capacité qu'ont ces acides sulfoniques spécifiques, même sous leur forme acide, de filtrer le rayonnement solaire.

Les acides sulfoniques pouvant être mis en oeuvre dans le cadre de la présente invention peuvent être représentés par la formule générale (a) :

$$R\text{-}SO_3H \qquad (a)$$

dans laquelle R représente un reste hydrocarboné aliphatique ou aromatique, éventuellement substitué.

Selon un mode de réalisation particulier de l'invention, R est choisi parmi le groupe comprenant les radicaux alkyle ayant de 1 à 30 atomes de carbone environ et alcényle ayant de 2 à 30 atomes de carbone environ, linéaires ou ramifiés et éventuellement substitués par un ou plusieurs groupements hydroxyle, alcoxy, acyloxy, aryle, cycloalkyle ou polycycloalkyle.

On peut citer comme exemples de tels composés :

l'acide méthane sulfonique $CH_3SO_3H$,
l'acide éthane sulfonique $CH_3CH_2SO_3H$,
l'acide n-butane 1-sulfonique $CH_3CH_2CH_2CH_2SO_3H$,
l'acide n-dodécane 1-sulfonique $CH_3\text{-}(CH_2)_{11}\text{-}SO_3H$,
l'acide n-octadécane 1-sulfonique $CH_3\text{-}(CH_2)_{17}\text{-}SO_3H$,
l'acide vinyl sulfonique $CH_2\text{=}CHSO_3H$,
l'acide 2-hydroxyéthane 1-sulfonique $HO\text{-}CH_2CH_2SO_3H$,
l'acide 4-hydrobutane 1-sulfonique $HO\text{-}(CH_2)_4SO_3H$,
l'acide campho-10-sulfonique.

Selon un autre mode de réalisation particulier de l'invention, R est un reste aryle ayant de 6 à 10 atomes de carbone éventuellement substitué par un ou plusieurs groupements hydroxyle, alcoxy, alcényloxy, alkyle, acyloxy, aryle, aryloxy, aroyle, aroyloxy, carboxyle, halogèno ou sulfonyle.

On peut citer comme exemple de tels composés :

l'acide benzène sulfonique :

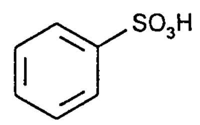

l'acide 4-méthyl benzène sulfonique :

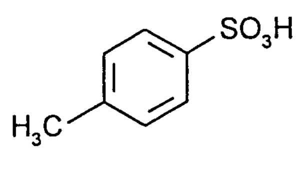

l'acide dodécyl benzène sulfonique (mélange d'isomères) :

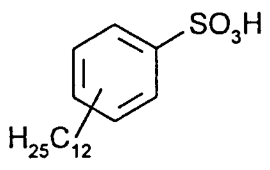

l'acide 5-sulfo salicylique :

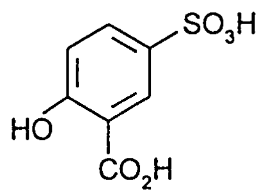

l'acide 5-sulfo isophtalique :

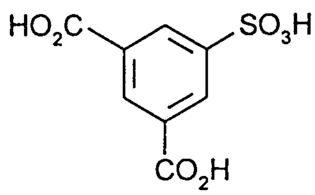

l'acide 4-méthoxy benzène sulfonique :

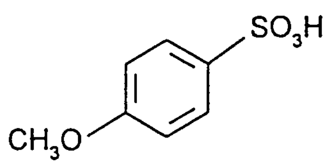

l'acide xylène sulfonique (mélange d'isomères) :

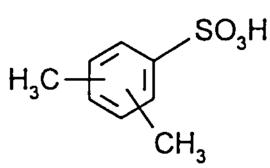

l'acide 3,6-dihydroxynaphtalène 2,7-disulfonique :

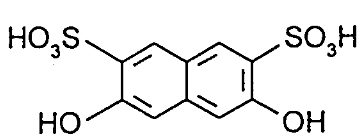

l'acide 1,8-dihydroxynaphtalène 3,6-disulfonique :

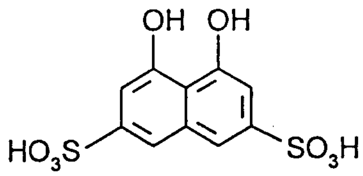

l'acide 4-chloro benzène sulfonique :

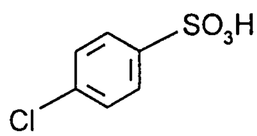

Selon un autre mode de réalisation particulier de l'invention, on met en oeuvre des dérivés sulfoniques ayant de plus la propriété de filtrer le rayonnement UV.

On peut citer comme exemples de tels composés ceux ayant la formule générale (b) suivante :

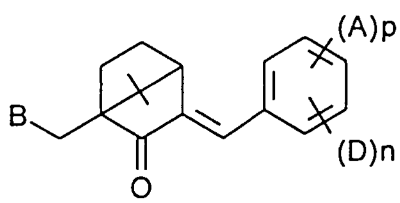

(b)

dans laquelle :

B représente H ou $SO_3H$

$0 \leq p \leq 1$ avec B = $SO_3H$ quand p = 0

$0 \leq n \leq 4$

D représente un ou plusieurs radicaux alkyle ou alcoxy, identiques ou différents quand $n \geq 2$, linéaires ou ramifiés contenant de 1 à 18 atomes de carbone environ, un radical halogéno, hydroxyle.

A, de préférence en méta ou en para, représente
soit un radical $SO_3H$
soit un groupement :

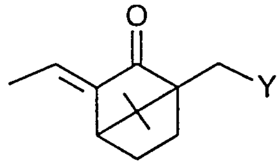

dans lequel Y représente H ou $SO_3H$

soit un groupement :

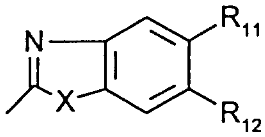

dans lequel :

$R_{11}$ désigne un atome d'hydrogène, un radical alkyle ou alcoxy, linéaire ou ramifié contenant de 1 à 6 atomes de carbone environ ou le radical $SO_3H$, $R_{11}$ étant $SO_3H$ lorsque B = H,

$R_{12}$ désigne un atome d'hydrogène, un radical alkyle ou alcoxy, linéaire ou ramifié, contenant de 1 à 6 atomes de carbone environ,

et X est un atome d'oxygène, de soufre ou un groupement -NR-, R étant un atome d'hydrogène ou un radical alkyle, linéaire ou ramifié, contenant de 1 à 6 atomes de carbone environ.

On peut citer comme exemples particuliers de composés de formule (b) les dérivés de formules (I), (II), (III) suivantes :

*Formule (I) :*

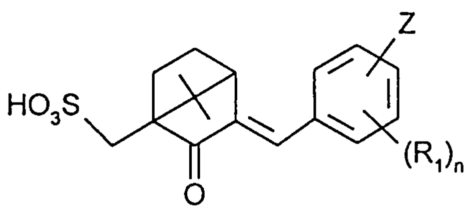

(I)

dans laquelle :

- Z, de préférence en position para ou méta, désigne un groupement

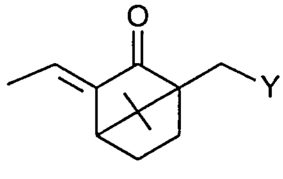

dans lequel Y représente H ou $SO_3H$

- n est égal à 0 ou est un nombre entier allant de 1 à 4 ($0 \leq n \leq 4$)
- $R_1$, représente un ou plusieurs radicaux alkyle ou alkoxy, identiques ou différents, linéaires ou ramifiés, contenant de 1 à 4 atomes de carbone environ.

Un composé de formule I particulièrement préféré est celui correspondant à n = 0, Z en position para et Y = $SO_3H$ : l'acide benzène 1,4 [di(3-méthylidènecampho-10-sulfonique)].

*Formule (II) :*

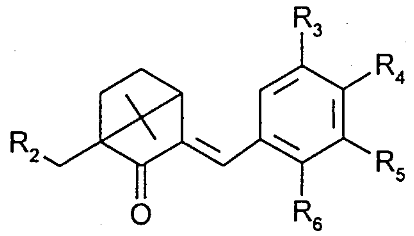

(II)

dans laquelle :

- $R_2$ désigne un atome d'hydrogène ou un radical $-SO_3H$ ;
- $R_3$, $R_4$, $R_5$ et $R_6$, identiques ou différents, représentent un groupement hydroxyle, un radical alkyle ayant de 1 à 4 atomes de carbone environ, linéaire ou ramifié, un radical alcényle ayant de 2 à 4 atomes de carbone environ, linéaire ou ramifié, un radical alcoxy ayant de 1 à 4 atomes de carbone, linéaire ou ramifié, un radical alcényloxy ayant de 2 à 4 atomes de carbone, linéaire ou ramifié, un radical halogéno; de plus un radical $R_3$ à $R_6$ seulement peut être un radical $SO_3H$, au moins un des radicaux $R_3$ à $R_6$ désignant le radical $-SO_3H$ quand $R_2$ est un atome d'hydrogène.

On peut citer comme exemples particuliers les composés suivants de formule II dans laquelle :

- $R_4$ désigne le radical $-SO_3H$ en position para du benzylidènecamphre et $R_2$, $R_3$, $R_5$ et $R_6$ désignent chacun un atome d'hydrogène, c'est-à-dire l'acide 4'-sulfo 3-benzylidènecamphre.
- $R_3$, $R_4$, $R_5$ et $R_6$ désignent chacun un atome d'hydrogène et $R_2$ désigne un radical $SO_3H$, c'est-à-dire l'acide 3-benzylidène campho-10-sulfonique.
- $R_4$ désigne un radical méthyle en position para du benzylidènecamphre, $R_5$ un radical $-SO_3H$ et $R_2$, $R_3$ et $R_6$ représentent un atome d'hydrogène, c'est-à-dire l'acide 4'-méthyl 3'-sulfo 3-benzylidènecamphre.
- $R_4$ désigne un atome de chlore en position para du benzylidènecamphre, $R_5$ un radical $-SO_3H$ et $R_2$, $R_3$ et $R_6$ représentent un atome d'hydrogène, c'est-à-dire l'acide 4'-chloro 3'-sulfo 3-benzylidènecamphre.
- $R_4$ désigne un radical méthyle en position para du benzylidènecamphre, $R_3$, $R_5$ et $R_6$ désignent un atome d'hydrogène et $R_2$ désigne un radical $-SO_3H$, c'est-à-dire l'acide 4'-méthyl 3-benzylidène campho-10-sulfonique.
- $R_2$ représente un radical $SO_3H$, $R_3$ est un radical méthyle, $R_4$ un atome d'hydrogène, $R_5$ un radical tertiobutyle, $R_6$ un radical hydroxyle, c'est-à-dire l'acide 3-(3-t-butyl 2-hydroxy 5-méthyl) benzylidène campho-10-sulfonique.
- $R_2$ représente un radical $SO_3H$, $R_3$ est un radical méthoxy, $R_4$ un atome d'hydrogène, $R_5$ un radical tertiobutyle, $R_6$ un radical hydroxyle, c'est-à-dire l'acide 3-(3-t-butyl 2-hydroxy 5-méthoxy) benzylidène campho-10-sulfonique.
- $R_2$ représente un radical $SO_3H$, $R_3$ et $R_5$ désignent chacun un radical tertiobutyle, $R_4$ un radical hydroxyle, $R_6$ un atome d'hydrogène, c'est-à-dire l'acide 3-(3,5-diterbutyl 4-hydroxy) benzylidène campho-10-sulfonique.
- $R_4$ représente un radical méthoxy en para, $R_5$ représente $SO_3H$, les radicaux $R_2$, $R_3$ et $R_6$ représentent H, c'est-à-dire l'acide 4'-méthoxy 3'-sulfo 3-benzylidènecamphre.
- $R_2$ désigne un radical $-SO_3H$, $R_3$ et $R_6$ représentent H, $R_4$ et $R_5$ formant un radical méthylènedioxy, c'est-à-dire l'acide 3-(4,5-méthylènedioxy) benzylidène campho-10-sulfonique.
- $R_2$ représente un radical $-SO_3H$, $R_4$ un radical méthoxy et les radicaux $R_3$,$R_5$ et $R_6$ représentent H, c'est-à-dire l'acide 3-(4-méthoxy) benzylidène campho-10-sulfonique.
- $R_2$ représente un radical $-SO_3H$, $R_4$ et $R_5$ sont tous deux un radical méthoxy et les radicaux $R_3$ et $R_6$ représentent H, c'est-à-dire l'acide 3-(4,5-diméthoxy) benzylidène campho-10-sulfonique.
- $R_2$ représente un radical $-SO_3H$, $R_4$ est un radical n-butoxy et les radicaux $R_3$,$R_5$ et $R_6$ représentent un atome d'hydrogène, c'est-à-dire l'acide 3-(4-n.butoxy) benzylidène campho-10-sulfonique.
- $R_2$ représente un radical $-SO_3H$, $R_4$ est un radical n-butoxy, $R_5$ est un radical méthoxy et $R_3$ et $R_6$ désignent tous deux un atome d'hydrogène, c'est-à-dire l'acide 3-(4-n.butoxy 5-méthoxy) benzylidène campho-10-sulfonique

*Formule (III) :*

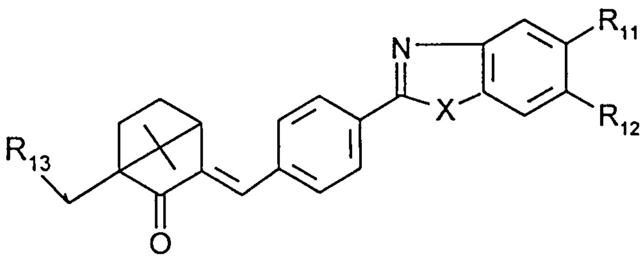

(III)

dans laquelle :

- $R_{11}$ désigne un atome d'hydrogène, un radical alkyle ou alcoxy, linéaire ou ramifié, contenant de 1 à 6 atomes de carbone environ ou un radical $-SO_3H$,
- $R_{12}$ désigne un atome d'hydrogène, un radical alkyle ou alcoxy, linéaire ou ramifié, contenant de 1 à 6 atomes de carbone environ,
- $R_{13}$ désigne un atome d'hydrogène ou un radical $-SO_3H$,
- l'un au moins des radicaux $R_{11}$ et $R_{13}$ désignant un radical $-SO_3H$,
- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer comme exemple particulier de formule (III) : le composé dans lequel X désigne un radical -NH-, $R_{11}$ désigne un radical $-SO_3H$, $R_{12}$ et $R_{13}$ désignent tous deux un atome d'hydrogène, c'est-à-dire l'acide 2-[4-(camphométhylidène) phényl] benzimidazole-5-sulfonique.

Les composés de structures (I), (II), (III) sont décrits dans le brevet US 4.585.597 et les brevets FR 2.236.515, 2.282.426, 2.645.148, 2.430.938, 2.592.380.

On peut citer comme autres exemples de composés ayant également la propriété de filtrer le rayonnement UV les composés de formule générale (c) suivante :

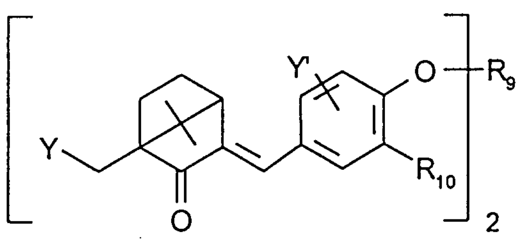

(c)

dans laquelle :

- $R_9$ désigne un radical divalent : $-(CH_2)_m-$ ou $-CH_2-CHOH-CH_2-$, m étant un nombre entier compris entre 1 et 10 ($1 \leq m \leq 10$),
- $R_{10}$ désigne un atome d'hydrogène, un radical alcoxy contenant de 1 à 4 atomes de carbone environ,
- Y et Y' désignent un atome d'hydrogène ou un radical $-SO_3H$, au moins un de ces radicaux Y ou Y' est différent de l'hydrogène.

On peut citer comme exemples particuliers les composés suivants de formule (c) dans laquelle Y représente $SO_3H$, Y' est H, R10 est H et R9 est $-CH_2-CH_2-$, c'est à dire l'acide éthylène bis [3(4'-oxy benzylidène) campho-10-sulfonique].

On peut encore citer comme exemples de dérivés sulfoniques ayant de plus la propriété de filtre du rayonnement UV les composés de formule générale (d) suivante :

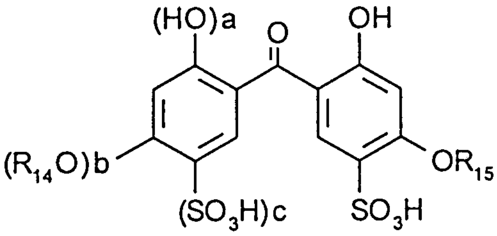

(d)

dans laquelle :

- $R_{14}$ et $R_{15}$, identiques ou différents, désignent soit un atome d'hydrogène soit un radical alkyle, linéaire ou ramifié, contenant de 1 à 8 atomes de carbone environ.
- a, b et c, identiques ou différents, sont égaux à 0 ou 1.

On peut citer comme exemple particulier de composé de formule (d) : l'acide 2-hydroxy 4-méthoxybenzophénone 5-sulfonique (composé de formule (d) dans laquelle a, b, et c sont égaux à zéro, et $R_{15}$ désigne un radical méthyle), désigné dans le dictionnaire CTFA par benzophénone-4.

Un autre exemple de dérivés sulfoniques ayant également la propriété de filtrer le rayonnement UV a la formule générale (e) :

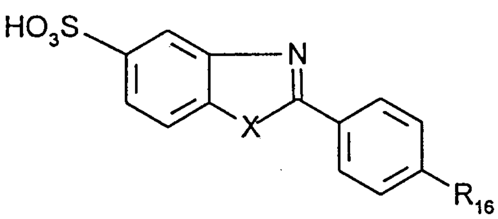

(e)

dans laquelle :

- X désigne un atome d'oxygène ou un radical -NH-
- $R_{16}$ désigne un atome d'hydrogène, un radical alkyle ou alcoxy, linéaire ou ramifié, contenant de 1 à 8 atomes de carbone environ ou un groupement de formule

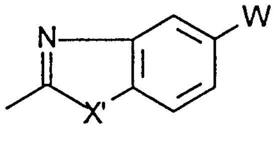

(IV)

dans laquelle X' représente un atome d'oxygène ou un radical -NH- et W représente H ou $SO_3H$.

On peut citer comme exemples particuliers les composés suivants de formule (e) dans laquelle:

- X désigne le radical -NH- et $R_{16}$ désigne un atome d'hydrogène : l'acide 2-phénylbenzimidazole 5-sulfonique, désigné par "2-phenylbenzimidazole 5-sulfonic acid" dans le dictionnaire CTFA.
- X désigne le radical -NH-, $R_{16}$ désigne le groupement de formule (IV) dans lequel W représente $SO_3H$ et X' dési-

gne le radical -NH- : l'acide benzène 1,4 - di(benzimidazol -2 yl-5-sulfonique).

- X désigne un atome d'oxygène, $R_{16}$ désigne le groupement de formule (IV) dans lequel W représente $SO_3H$ et X' désigne un atome d'oxygène : l'acide benzène 1,4-di(benzoxazol -2 yl-5-sulfonique).

Les composés de formule (d) et (e) sont des composés connus pouvant être préparés selon des méthodes classiques décrites dans l'art antérieur.

Les acides sulfoniques sont utilisés, selon la présente invention, en quantité de préférence allant de 0,01 à 15 % en poids et encore plus préférentiellement de 0,5 à 10 %.

Si les acides sulfoniques selon l'invention sont partiellement neutralisés, c'est, comme indiqué plus haut, en une quantité insuffisante pour neutraliser toutes les fonctions acides sulfoniques présentes dans la composition. La neutralisation est obtenue de façon classique à l'aide d'une base minérale ou organique comme par exemple la soude, la potasse, l'ammoniaque, la monoéthanolamine, la triéthanolamine, l'isopropanol-amine, les acides aminés, etc.

Les compositions cosmétiques selon l'invention peuvent contenir un ou plusieurs filtres solaires actifs dans l'UVA et/ou l'UVB, hydrophiles ou lipophiles, ne présentant pas de fonction sulfonique. Ces filtres peuvent être des filtres chimiques ou des pigments.

Les compositions de l'invention peuvent comprendre en outre les adjuvants classiquement mis en oeuvre dans les domaines considérés comme les corps gras, les solvants organiques, les épaississants ioniques ou non ioniques, les adoucissants, les antioxydants, les opacifiants, les stabilisants, les silicones, les agents anti-mousse, les agents hydratants, les vitamines, les parfums, les tensioactifs ioniques ou non, les charges, les séquestrants, les colorants ou tout autre ingrédient habituellement utilisé en cosmétique.

Les compositions de l'invention peuvent comprendre aussi des actifs susceptibles de compléter l'effet des actifs anti-acnéiques selon l'invention, et notamment des agents anti-inflammatoires tels que le peroxyde de benzoyle, des antibiotiques, des agents antiseptiques tels que l'octopirox ou des agents antiséborrhéiques.

Les compositions selon l'invention sont préparées selon les techniques bien connues de l'homme de l'art du domaine.

Les compositions selon l'invention peuvent se présenter sous forme de suspension ou de dispersion dans des solvants ou des corps gras, sous forme de dispersion vésiculaire ou encore sous forme d'émulsion telle qu'une crème ou un lait, sous forme de pommade, de gel, de bâtonnet solide, de mousse aérosol ou de spray.

Les exemples suivants illustrent l'invention sans en limiter aucunement la portée. Les pourcentages sont donnés en poids.

**Exemple 1 : Crème**

| | |
|---|---|
| - Glycérine | 3 % |
| - Huile végétale | 4 % |
| - Acide benzène 1,4[di(méthylidène campho-10-sulfonique)] | 2 % |
| - Huile de silicone volatile | 5 % |
| - Polymère carboxyvinylique (Carbopol 980) | 0,9 % |
| - Soude qsp pH 4 | |
| - Eau déminéralisée | qsp 100% |

La composition obtenue a un pH d'environ 4 et se présente sous forme d'une crème apte à traiter l'acné.

**Exemple 2 : Crème**

| | |
|---|---|
| - Glycérine | 3 % |
| - Fraction liquide de beurre de karité | 2 % |
| - Polisobutène hydrogéné | 10 % |
| - Stéarate de PEG-40 | 2 % |
| - Stéarate de glycéryle | 3 % |
| - Alcool cétylique | 4 % |
| - Acide benzène 1,4[di(méthylidène campho-10-sulfonique)] | 0,7 % |
| - Huile de silicone volatile | 5 % |
| - Polyacrylamide/C13-C16 Isoparaffine/laureth-7 (Sepigel 305 vendu par la société Seppic) | 1 % |
| - EDTA disodique | 0,5 % |
| - Conservateurs | 0,2 % |
| - Eau déminéralisée | qsp 100% |

La composition obtenue a un pH d'environ 3 et se présente sous forme d'une crème apte à traiter l'acné.

Le test ci-dessous met en évidence l'effet kératolytique de l'acide benzène 1,4[di(méthylidène campho-10-sulfonique)] comparé à l'acide lactique et à un dérivé de l'acide salicylique, connus comme actifs anti-acnéiques. Le support des formules utilisées était proche de celui de l'exemple 2.

Le test consiste à évaluer *in vivo* l'effet kératolytique de ces actifs par visualisation de la couche cornée au moyen du nitrate d'argent. Le noircissement permet de suivre la desquamation des zones traitées et d'une zone témoin par mesure de la luminance (L*) grâce au chromamètre CR300.

Pour réaliser le test, on applique sur une zone délimitée 4 mg/cm$^2$ de peau, d'une composition contenant l'actif. Une heure après l'application, on rince et essuie la zone testée, puis on applique 20 µl d'une solution à 1 % de nitrate d'argent dans l'eau et, après séchage, on dépose 40 µl de la solution de révélateur pour transformer en argent le nitrate d'argent.

On mesure la luminance avant application ($L^*_{T0}$) et après une heure d'application ($L^*_{T1}$), pour la zone traitée et pour la zone témoin.

Le pourcentage d'effet kératolytique est évalué selon l'équation suivante :

$$\% \text{ desquamation} = 100 \times \frac{(L^*_{T1} - L^*_{T0})_{\text{traitée}} - (L^*_{T1} - L^*_{T0})_{\text{témoin}}}{(L^*_{T1} - L^*_{T0})_{\text{témoin}}}$$

Les compositions testées étaient les suivantes :

A. Composition à 1 % d'acide n-octanoyl-5 salicylique
B. Composition à 0,7 % d'acide benzène 1,4[di(méthylidène campho-10-sulfonique)] à pH 1,9
C. Composition à 0,7 % d'acide benzène 1,4[di(méthylidène campho-10-sulfonique)] à pH 4,5
D. Composition à 5,5 % d'acide lactique.

| Composition | % effet kératolytique |
|---|---|
| A | 67 % |
| B selon l'invention | 91 % |
| C selon l'invention | 63 % |
| D | 15 % |

Ces résultats mettent en évidence l'effet kératolytique de l'acide benzène 1,4[di(méthylidène campho-10-sulfonique)] selon l'invention, notamment à pH acide (composition B).

## Revendications

1. Utilisation d'au moins un composé présentant au moins une fonction acide sulfonique non complètement neutralisée, comme actif anti-acnéique dans ou pour la fabrication d'une composition topique.

2. Utilisation d'au moins un composé présentant au moins une fonction acide sulfonique non complètement neutralisée, dans ou pour la fabrication d'une composition topique pour traiter la rétention séborrhéique et l'hyperproduction de sébum de la peau.

3. Utilisation selon la revendication 1 ou 2, caractérisée en ce que la composition a un pH inférieur à 6.

4. Utilisation selon la revendication 3, caractérisée en ce que la composition a un pH allant de 1 à 5.

5. Utilisation selon l'une quelconque des revendications précédentes, caractérisée en ce que le dit composé a pour formule générale

$$RSO_3H \qquad (a)$$

dans laquelle R représente un radical hydrocarboné aliphatique ou aromatique.

6. Utilisation selon la revendication 5, caractérisée en ce que R est un reste hydrocarboné aliphatique choisi parmi le groupe comprenant les radicaux alkyle ayant de 1 à 30 atomes de carbone et alcényle ayant de 2 à 30 atomes de carbone, linéaires ou ramifiés, éventuellement substitués par un ou plusieurs groupements hydroxyle, alcoxy, acyloxy, aryle, cycloalkyle ou polycycloalkyle.

7. Utilisation selon la revendication 5 ou 6, caractérisée en ce que R est le radical $CH_3$ ou $CH_3$-$(CH_2)_{11}$.

8. Utilisation selon les revendications 5 ou 6, caractérisée en ce que le dit composé est l'acide campho-10-sulfonique.

9. Utilisation selon la revendication 5, caractérisée en ce que R est un reste hydrocarboné aromatique ayant de 6 à 10 atomes de carbone éventuellement substitué par un ou plusieurs groupements hydroxyle, alcoxy, alcényloxy, alkyle, acyloxy, aryle, aryloxy, aroyle, aroyloxy, carboxyle, halogèno ou sulfonyle.

10. Utilisation selon la revendication 9, caractérisée en ce que R est le radical $C_6H_5$.

11. Utilisation selon la revendication 10, caractérisée en ce que R est le radical

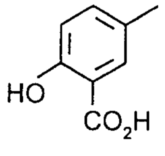

**12.** Utilisation selon la revendication 5, caractérisée en ce que le dit composé a pour formule générale :

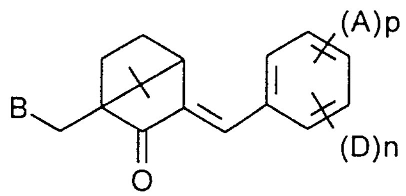

(b)

dans laquelle :

B représente H ou $SO_3H$

$0 \leq p \leq 1$ avec B = $SO_3H$ quand p = 0

$0 \leq n \leq 4$

D représente un ou plusieurs radicaux alkyle ou alcoxy, identiques ou différents linéaires ou ramifiés contenant de 1 à 18 atomes de carbone environ, halogéno, hydroxyle

A, de préférence en méta ou en para, représente
soit un radical $SO_3H$
soit un groupement

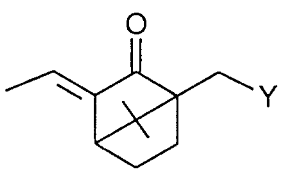

dans lequel Y représente H ou $SO_3H$
soit un groupement

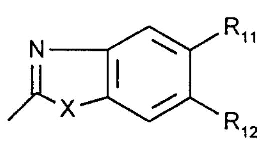

dans lequel

$R_{11}$ désigne un atome d'hydrogène, un radical alkyle ou alcoxy, linéaire ou ramifié contenant de 1 à 6 atomes de carbone environ ou le radical $SO_3H$, $R_{11}$ étant $SO_3H$ lorsque B = H,

$R_{12}$ désigne un atome d'hydrogène, un radical alkyle ou alcoxy, linéaire ou ramifié, contenant de 1 à 6 atomes de carbone environ,

et X est un atome d'oxygène, de soufre ou un groupement -NR-, R étant un atome d'hydrogène ou un radical alkyle, linéaire ou ramifié, contenant de 1 à 6 atomes de carbone environ.

**13.** Utilisation selon la revendication 12, caractérisée en ce que le composé de formule (b) a pour formule :

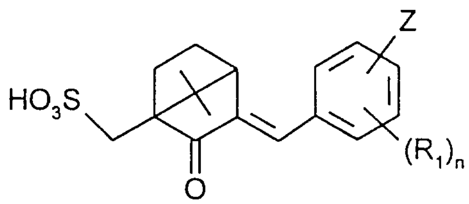

(I)

dans laquelle :

- Z, de préférence en position para ou méta, désigne un groupement

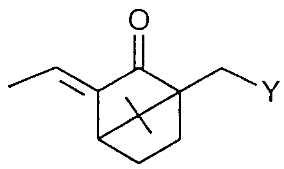

dans lequel Y représente H ou $SO_3H$

- n est égal à 0 ou est un nombre entier allant de 1 à 4 ($0 \leq n \leq 4$)
- $R_1$, représente un ou plusieurs radicaux alkyle ou alkoxy, identiques ou différents, linéaires ou ramifiés, contenant de 1 à 4 atomes de carbone environ.

**14.** Utilisation selon la revendication 13, caractérisée en ce que le composé de formule (I) est l'acide benzène 1,4 [di(3-méthylidènecampho 10-sulfonique)].

**15.** Utilisation selon la revendication 12, caractérisée en ce que le composé de formule (b) a pour formule :

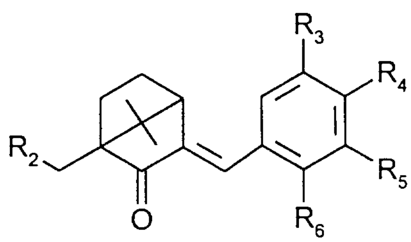

(II)

dans laquelle :

- $R_2$ désigne un atome d'hydrogène ou un radical $-SO_3H$,
- $R_3$, $R_4$, $R_5$ et $R_6$, identiques ou différents, représentent un groupement hydroxyle, un radical alkyle ayant de 1 à 4 atomes de carbone, linéaire ou ramifié, un radical alcényle ayant de 2 à 4 atomes de carbone, linéaire ou ramifié, un radical alcoxy ayant de 1 à 4 atomes de carbone, linéaire ou ramifié, un radical alcényloxy ayant de 2 à 4 atomes de carbone, linéaire ou ramifié, un radical halogéno, un radical $R_3$ à $R_6$ seulement pouvant être un radical $SO_3H$, au moins un des radicaux $R_3$ à $R_6$ étant le radical $-SO_3H$ quand $R_2$ est un atome d'hydrogène.

**16.** Utilisation selon la revendication 15, caractérisée en ce que le composé de formule (II) est choisi parmi l'acide 4'-sulfo 3-benzylidènecamphre, l'acide 3-benzylidène campho-10 sulfonique, l'acide 4'-méthyl 3'-sulfo 3-benzylidènecamphre, l'acide 4'-chloro 3'-sulfo 3-benzylidènecamphre, l'acide 4'-méthyl 3-benzylidène campho-10-sulfonique, l'acide 3-(3-t-butyl 2-hydroxy 5-méthyl) benzylidène campho-10-sulfonique, l'acide 3-(3-t-butyl 2-hydroxy 5-méthoxy) benzylidène campho-10-sulfonique, l'acide 3-(3,5-diterbutyl 4-hydroxy) benzylidène campho-10-sulfonique, l'acide 4'-méthoxy 3'-sulfo 3-benzylidènecamphre, l'acide 3-(4,5-méthylènedioxy) benzylidène campho-10-sulfonique, l'acide 3-(4-méthoxy) benzylidène campho-10-sulfonique, l'acide 3-(4,5-diméthoxy) benzylidène campho-10-sulfonique, l'acide 3-(4-n. butoxy) benzylidène campho-10-sulfonique, ou l'acide 3-(4-n.butoxy 5-méthoxy) benzylidène campho-10-sulfonique.

**17.** Utilisation selon la revendication 12, caractérisée en ce que le composé de formule (b) a pour formule :

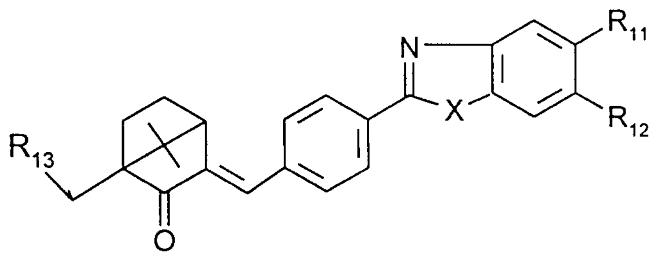

(III)

dans laquelle :

- $R_{11}$ désigne un atome d'hydrogène, un radical alkyle ou alcoxy, linéaire ou ramifié, contenant de 1 à 6 atomes de carbone environ ou un radical $-SO_3H$,
- $R_{12}$ désigne un atome d'hydrogène, un radical alkyle ou alcoxy, linéaire ou ramifié, contenant de 1 à 6 atomes de carbone environ,
- $R_{13}$ désigne un atome d'hydrogène ou un radical $-SO_3H$,
- l'un au moins des radicaux $R_{11}$ et $R_{13}$ désignant un radical $-SO_3H$,
- X est un atome d'oxygène ou de soufre ou un groupement -NR-, R étant un atome d'hydrogène ou un radical alkyle, linéaire ou ramifié, contenant de 1 à 6 atomes de carbone environ.

**18.** Utilisation selon la revendication 17, caractérisée en ce que le composé de formule (III) est l'acide 2-[4-(camphométhylidène) phényl] benzimidazole-5-sulfonique.

**19.** Utilisation selon la revendication 5, caractérisée en ce que ledit composé a pour formule :

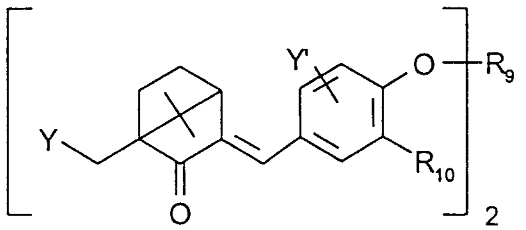

(c)

dans laquelle :

- $R_9$ désigne un radical divalent : $-(CH_2)_m-$ ou $-CH_2$ $-CHOH-CH_2-$, m étant un nombre entier compris entre 1 et 10 ($1 \leq m \leq 10$),
- $R_{10}$ désigne un atome d'hydrogène, un radical alcoxy contenant de 1 à 4 atomes de carbone environ,
- Y et Y' désignent un atome d'hydrogène ou un radical $-SO_3H$, au moins un de ces radicaux Y ou Y' étant différent de l'hydrogène.

**20.** Utilisation selon la revendication 19, caractérisée en ce que le composé de formule (c) est l'acide éthylène bis [3-(4'-oxy benzylidène) campho-10-sulfonique].

**21.** Utilisation selon la revendication 5, caractérisée en ce que ledit composé a pour formule :

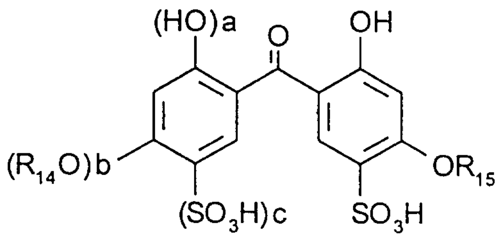

(d)

dans laquelle :

- $R_{14}$ et $R_{15}$, identiques ou différents, désignent soit un atome d'hydrogène soit un radical alkyle, linéaire ou ramifié, contenant de 1 à 8 atomes de carbone environ.
- a, b et c, identiques ou différents, sont égaux à 0 ou 1.

**22.** Utilisation selon la revendication 21, caractérisée en ce que le composé de formule (d) est l'acide 2-hydroxy 4-méthoxybenzophénone 5-sulfonique.

**23.** Utilisation selon la revendication 5, caractérisée en ce que le dit composé a pour formule générale :

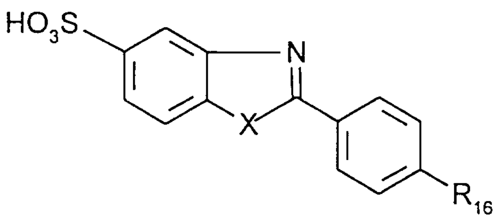

(e)

dans laquelle :

- X désigne un atome d'oxygène ou un radical -NH-
- $R_{16}$ désigne un atome d'hydrogène, un radical alkyle ou alcoxy, linéaire ou ramifié, contenant de 1 à 8 atomes de carbone environ ou un groupement de formule

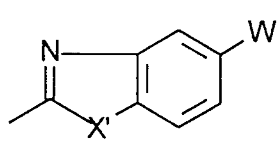

(IV)

dans laquelle X' représente un atome d'oxygène ou un radical -NH- et W représente H ou $SO_3H$.

**24.** Utilisation selon la revendication 23, caractérisée en ce que le composé de formule (e) est l'acide 2-phénylbenzimidazole 5-sulfonique, l'acide benzène 1,4-di(benzimidazol 2 yl 5-sulfonique) ou l'acide benzène 1,4-di(benzoxazol-2 yl -5-sulfonique).

**25.** Utilisation selon l'une quelconque des revendications précédentes, caractérisée en ce que le dit composé est mis en oeuvre en une quantité allant de 0,01 à 15 % en poids de la composition et plus préférentiellement allant de 0,5 à 10 %.

**26.** Procédé de traitement cosmétique de l'acné consistant à appliquer sur la peau un composé présentant au moins

une fonction acide sulfonique non complètement neutralisée.

## Claims

1. Use of at least one compound having at least one sulphonic acid function which is not completely neutralized, as anti-acne active agent in, or for the manufacture of, a topical composition.

2. Use of at least one compound having at least one sulphonic acid function which is not completely neutralized, in, or for the manufacture of, a topical composition for treating seborrhoeic retention and the overproduction of sebum in the skin.

3. Use according to Claim 1 or 2, characterized in that the composition has a pH of less than 6.

4. Use according to Claim 3, characterized in that the composition has a pH ranging from 1 to 5.

5. Use according to any one of the preceding claims, characterized in that the said compound has the general formula

$$RSO_3H \quad (a)$$

in which R represents an aliphatic or aromatic hydrocarbon radical.

6. Use according to Claim 5, characterized in that R is an aliphatic hydrocarbon residue chosen from the group comprising alkyl radicals having from 1 to 30 carbon atoms and alkenyl radicals having from 2 to 30 carbon atoms, which are linear or branched and optionally substituted with one or more hydroxyl, alkoxy, acyloxy, aryl, cycloalkyl or polycycloalkyl groups.

7. Use according to Claim 5 or 6, characterized in that R is the $CH_3$ or $CH_3$-$(CH_2)_{11}$ radical.

8. Use according to Claims 5 or 6, characterized in that the said compound is 10-camphorsulphonic acid.

9. Use according to Claim 5, characterized in that R is an aromatic hydrocarbon residue having from 6 to 10 carbon atoms, optionally substituted with one or more hydroxyl, alkoxy, alkenyloxy, alkyl, acyloxy, aryl, aryloxy, aroyl, aroyloxy, carboxyl, halo or sulphonyl groups.

10. Use according to Claim 9, characterized in that R is the $C_6H_5$ radical.

11. Use according to Claim 10, characterized in that R is the radical

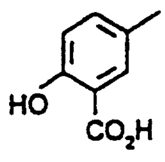

12. Use according to Claim 5, characterized in that the said compound has the general formula:

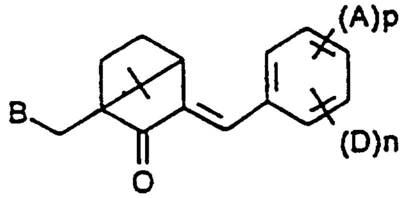

(b)

in which:

B represents H or $SO_3H$
$0 \leq p \leq 1$ with B = $SO_3H$ when p = 0
$0 \leq n \leq 4$
D represents one or more identical or different, linear or branched alkyl or alkoxy radicals containing from 1 to 18 carbon atoms approximately, halo or hydroxyl radicals
A, preferably in the meta or para position, represents either an $SO_3H$ radical
or a group

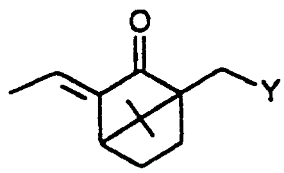

in which Y represents H or $SO_3H$
or a group

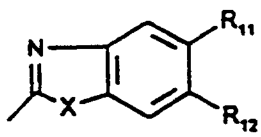

in which
$R_{11}$ denotes a hydrogen atom, a linear or branched alkyl or alkoxy radical containing from 1 to 6 carbon atoms approximately or the $SO_3H$ radical, $R_{11}$ being $SO_3H$ when B = H,
$R_{12}$ denotes a hydrogen atom or a linear or branched alkyl or alkoxy radical containing from 1 to 6 carbon atoms approximately,
and X is an oxygen or sulphur atom or a group -NR-, R being a hydrogen atom or a linear or branched alkyl radical containing from 1 to 6 carbon atoms approximately.

**13.** Use according to Claim 12, characterized in that the compound of formula (b) has the formula:

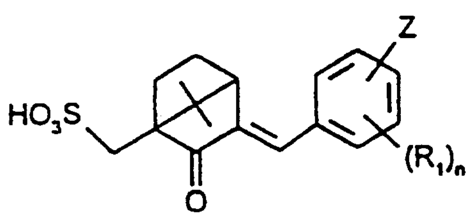

(I)

in which:

- Z, preferably in the meta or para position, denotes a group

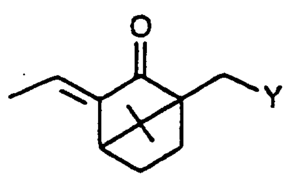

in which Y represents H or $SO_3H$
- n is equal to 0 or is an integer ranging from 1 to 4 ($0 \leq n \leq 4$)

- $R_1$ represents one or more identical or different, linear or branched alkyl or alkoxy radicals containing from 1 to 4 carbon atoms approximately.

**14.** Use according to Claim 13, characterized in that the compound of formula (I) is benzene-1,4-[di(3-methylidene-10-camphorsulphonic)] acid.

**15.** Use according to Claim 12, characterized in that the compound of formula (b) has the formula:

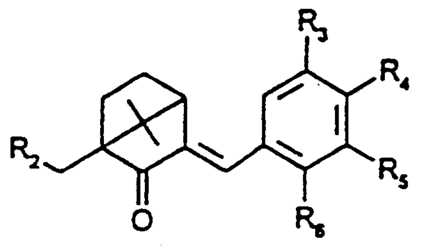

(II)

in which:

- $R_2$ denotes a hydrogen atom or an $-SO_3H$ radical,
- $R_3$, $R_4$, $R_5$ and $R_6$, which may be identical or different, represent a hydroxyl group, a linear or branched alkyl radical having from 1 to 4 carbon atoms, a linear or branched alkenyl radical having from 2 to 4 carbon atoms, a linear or branched alkoxy radical having from 1 to 4 carbon atoms, a linear or branched alkenyloxy radical having from 2 to 4 carbon atoms, a halo radical, one radical $R_3$ to $R_6$ only being able to be an $SO_3H$ radical, at least one of the radicals $R_3$ to $R_6$ being the $-SO_3H$ radical when $R_2$ is a hydrogen atom.

**16.** Use according to Claim 15, characterized in that the compound of formula (II) is chosen from 3-benzylidenecamphor-4'-sulphonic acid, 3-benzylidene-10-camphorsulphonic acid, 4'-methyl-3-benzylidenecamphor-3'-sulphonic acid, 4'-chloro-3-benzylidenecamphor-3'-sulphonic acid, 4'-methyl-3-benzylidene-10-camphorsulphonic acid, 3-(3-t-butyl-2-hydroxy-5-methyl)benzylidene-10-camphorsulphonic acid, 3-(3-t-butyl-2-hydroxy-5-methoxy)benzylidene-10-camphorsulphonic acid, 3-(3,5-di-tert-butyl-4-hydroxy)benzylidene-10-camphorsulphonic acid, 4'-methoxy-3-benzylidenecamphor-3'-sulphonic acid, 3-(4,5-methylenedioxy)benzylidene-10-camphorsulphonic acid, 3-(4-methoxy)benzylidene-10-camphorsulphonic acid, 3-(4,5-dimethoxy)benzylidene-10-camphorsulphonic acid, 3-(4-n-butoxy)benzylidene-10-camphorsulphonic acid or 3-(4-n-butoxy-5-methoxy)benzylidene-10-camphorsulphonic acid.

**17.** Use according to Claim 12, characterized in that the compound of formula (b) has the formula:

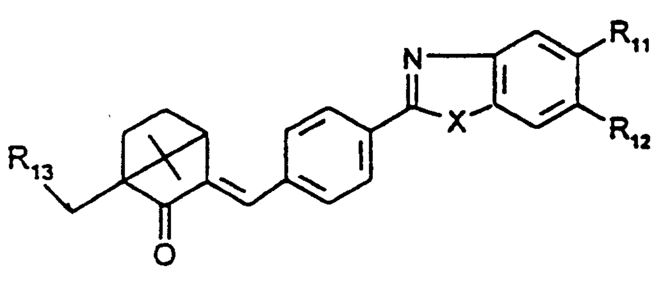

(III)

in which:

- $R_{11}$ denotes a hydrogen atom, a linear or branched alkyl or alkoxy radical containing from 1 to 6 carbon atoms approximately or an $-SO_3H$ radical,
- $R_{12}$ denotes a hydrogen atom or a linear or branched alkyl or alkoxy radical containing from 1 to 6 carbon atoms approximately,
- $R_{13}$ denotes a hydrogen atom or an $-SO_3H$ radical,
- at least one of the radicals $R_{11}$ and $R_{13}$ denoting an
- $SO_3H$ radical,
- X is an oxygen or sulphur atom or a group -NR-, R being a hydrogen atom or a linear or branched alkyl radical

containing from 1 to 6 carbon atoms approximately.

**18.** Use according to Claim 17, characterized in that the compound of formula (III) is 2-[4-(camphormethylidene)phenyl]benzimidazole-5-sulphonic acid.

**19.** Use according to Claim 5, characterized in that the said compound has the formula:

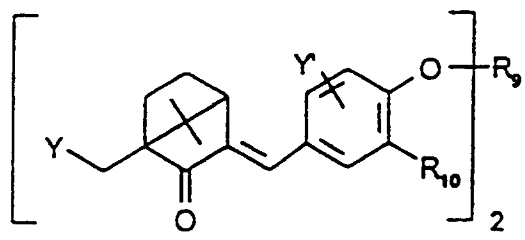

(c)

in which:

- $R_9$ denotes a divalent radical: $-(CH_2)_m-$ or $-CH_2-CHOH-CH_2-$, m being an integer between 1 and 10 ($1 \leq m \leq 10$),
- $R_{10}$ denotes a hydrogen atom or an alkoxy radical containing from 1 to 4 carbon atoms approximately,
- Y and Y' denote a hydrogen atom or an $-SO_3H$ radical, at least one of these radicals Y or Y' being other than hydrogen.

**20.** Use according to Claim 19, characterized in that the compound of formula (c) is ethylenebis[3-(4'-oxybenzylidene)-10-camphorsulphonic] acid.

**21.** Use according to Claim 5, characterized in that the said compound has the formula:

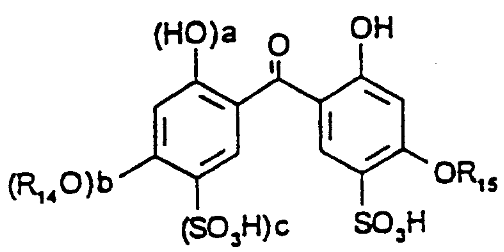

(d)

in which:

- $R_{14}$ and $R_{15}$, which may be identical or different, denote either a hydrogen atom or a linear or branched alkyl radical containing from 1 to 8 carbon atoms approximately,
- a, b and c, which may be identical or different, are equal to 0 or 1.

**22.** Use according to Claim 21, characterized in that the compound of formula (d) is 2-hydroxy-4-methoxybenzophenone-5-sulphonic acid.

**23.** Use according to Claim 5, characterized in that the said compound has the general formula:

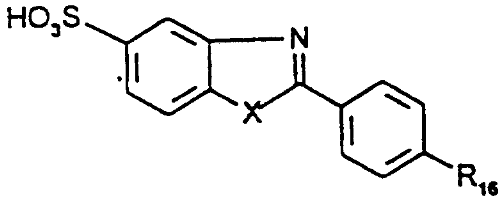

(e)

in which:

- X denotes an oxygen atom or an -NH- radical
- $R_{16}$ denotes a hydrogen atom, a linear or branched alkyl or alkoxy radical containing from 1 to 8 carbon atoms approximately or a group of formula

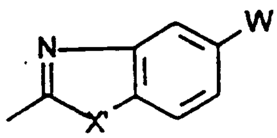

(IV)

in which X' represents an oxygen atom or an -NH- radical and W represents H or $SO_3H$.

**24.** Use according to Claim 23, characterized in that the compound of formula (e) is 2-phenylbenzimidazole-5-sulphonic acid, benzene-1,4-di(2-benzimidazolyl-5-sulphonic) acid or benzene-1,4-di(2-benzoxazolyl-5-sulphonic) acid.

**25.** Use according to any one of the preceding claims, characterized in that the said compound is used in an amount ranging from 0.01% to 15% of the weight of the composition and more preferably ranging from 0.5 to 10%.

**26.** Process for the cosmetic treatment of acne, which consists in applying to the skin a compound having at least one sulphonic acid function which is not completely neutralized.

## Patentansprüche

**1.** Verwendung mindestens einer Verbindung, die mindestens eine Sulfonsäuregruppe aufweist, wobei die Sulfongruppen nicht vollständig neutralisiert sind, als Wirkstoff gegen Akne in einer topischen Zusammensetzung oder zu deren Herstellung.

**2.** Verwendung mindestens einer Verbindung, die mindestens eine Sulfonsäuregruppe aufweist, wobei die Sulfongruppen nicht vollständig neutralisiert sind, zur Behandlung der Sebumretention und gesteigerten Sebumproduktion der Haut in einer topischen Zusammensetzung oder zu deren Herstellung.

**3.** Verwendung nach Anspruch 1 oder 2, dadurch gekennzeichnet, daß die Zusammensetzung einen pH-Wert unter 6 aufweist.

**4.** Verwendung nach Anspruch 3, dadurch gekennzeichnet, daß die Zusammensetzung einen pH-Wert im Bereich von 1 bis 5 aufweist.

**5.** Verwendung nach einem der vorhergehenden Ansprüche, dadurch gekennzeichnet, daß die Verbindung der allgemeinen Formel entspricht:

$$RSO_3H \qquad (a),$$

worin R eine aliphatische oder aromatische Kohlenwasserstoffgruppe bedeutet.

**6.** Verwendung nach Anspruch 5, dadurch gekennzeichnet, daß R eine aliphatische Kohlenwasserstoffgruppe ist, die

unter den Alkylgruppen mit 1 bis 30 Kohlenstoffatomen und den Alkenylgruppen mit 2 bis 30 Kohlenstoffatomen ausgewählt ist, die geradkettig oder verzweigt vorliegen und gegebenenfalls mit einer oder mehreren Gruppen Hydroxy, Alkoxy, Acyloxy, Aryl, Cycloalkyl oder Polycycloalkyl substituiert sind.

**7.** Verwendung nach Anspruch 5 oder 6, dadurch gekennzeichnet, daß R die Gruppe $CH_3$ oder $CH_3$-$(CH_2)_{11}$ bedeutet.

**8.** Verwendung nach Anspruch 5 oder 6, dadurch gekennzeichnet, daß die Verbindung die Campher-10-Sulfonsäure ist.

**9.** Verwendung nach Anspruch 5, dadurch gekennzeichnet, daß R eine aromatische Kohlenwasserstoffgruppe mit 6 bis 10 Kohlenstoffatomen bedeutet, die gegebenenfalls mit einer oder mehreren Gruppen Hydroxy, Alkoxy, Alkenyloxy, Alkyl, Acyloxy, Aryl, Aryloxy, Aroyl, Aroyloxy, Carboxy, Halogen oder Sulfonyl substituiert ist.

**10.** Verwendung nach Anspruch 9, dadurch gekennzeichnet, daß R die Gruppe $C_6H_5$ bedeutet.

**11.** Verwendung nach Anspruch 10, dadurch gekennzeichnet, daß R die Gruppe

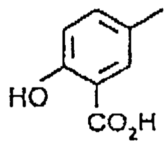

bedeutet.

**12.** Verwendung nach Anspruch 5, dadurch gekennzeichnet, daß die Verbindung die allgemeine Formel aufweist:

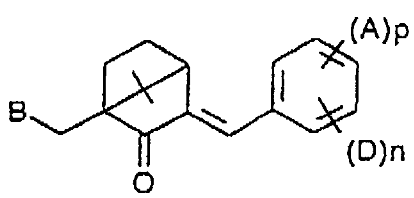

(b) ,

worin bedeuten:

- B, H oder $SO_3H$,
- $0 \leq p \leq 1$ mit B = $SO_3H$, wenn p = 0,
- $0 \leq n \leq 4$,
- D eine oder mehrere geradkettige oder verzweigte Alkyl- oder Alkoxygruppen mit etwa 1 bis 18 Kohlenstoffatomen, die identisch oder voneinander verschieden sind, Halogen oder Hydroxy,
- A, das sich vorzugsweise in m- oder p-Stellung befindet,

  - $SO_3H$
  - eine Gruppe:

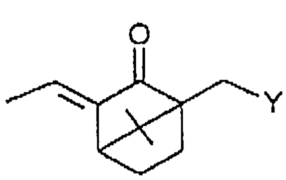

,

worin Y H oder $SO_3H$ bedeutet,

- eine Gruppe;

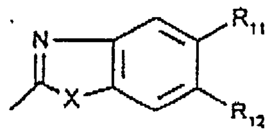

,

worin bedeuten:

$R_{11}$ Wasserstoff, eine geradkettige oder verzweigte Alkyl- oder Alkoxygruppe mit etwa 1 bis 6 Kohlenstoffatomen oder die Gruppe $SO_3H_4$ wobei $R_{11}$ $SO_3H$ bedeutet, wenn B H ist,

$R_{12}$ Wasserstoff oder eine geradkettige oder verzweigte Alkyl- oder Alkoxygruppe mit etwa 1 bis 6 Kohlenstoffatomen, und

X Sauerstoff, Schwefel oder eine Gruppe -NR-, wobei R ein Wasserstoffatom oder eine geradkettige oder verzweigte Alkylgruppe mit etwa 1 bis 6 Kohlenstoffatomen bedeutet.

**13.** Verwendung nach Anspruch 12, dadurch gekennzeichnet, daß die Verbindung der Formel (b) folgende Formel aufweist;

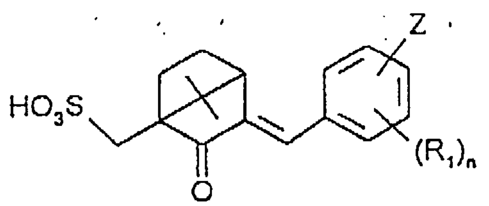

(I),

worin bedeuten:

- Z, das sich vorzugsweise in p- oder m-Stellung befindet, eine Gruppe

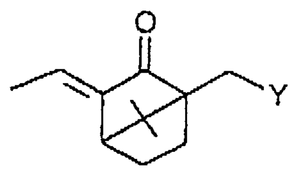

,

worin Y H oder $SO_3H$ bedeutet,

- n Null oder eine ganze Zahl im Bereich von 1 bis 4 ($0 \leq n \leq 4$),

- $R_1$ eine oder mehrere geradkettige oder verzweigte Alkyl- oder Alkoxygruppen mit etwa 1 bis 4 Kohlenstoffatomen, die identisch oder voneinander verschieden sind.

**14.** Verwendung nach Anspruch 13, dadurch gekennzeichnet, daß die Verbindung der Formel (I) die Benzol-1,4-di(3-methylidencampher-10-sulfonsäure) ist.

**15.** Verwendung nach Anspruch 12, dadurch gekennzeichnet, daß die Verbindung der Formel (b) folgende Formel aufweist;

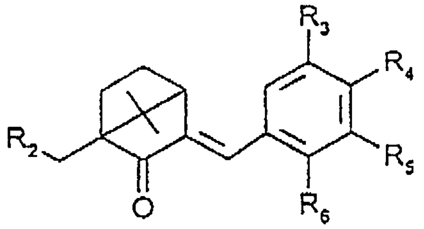

(II),

worin bedeuten:

- $R_2$ Wasserstoff oder eine Gruppe $-SO_3H$,

- $R_3$, $R_4$, $R_5$ und $R_6$, die identisch oder voneinander verschieden sind, Hydroxy, eine geradkettige oder verzweigte Alkylgruppe mit 1 bis 4 Kohlenstoffatomen, eine geradkettige oder verzweigte Alkenylgruppe mit 2 bis 4 Kohlenstoffatomen, eine geradkettige oder verzweigte Alkoxygruppe mit 1 bis 4 Kohlenstoffatomen, eine geradkettige oder verzweigte Alkenyloxygruppe mit 2 bis 4 Kohlenstoffatomen oder Halogen; eine der Gruppen $R_3$ bis $R_6$ kann eine Gruppe $-SO_3H$ bedeuten, wobei mindestens eine der Gruppe $R_3$ bis $R_6$ $SO_3H$ bedeutet, wenn $R_2$ ein Wasserstoffatom ist.

**16.** Verwendung nach Anspruch 15, dadurch gekennzeichnet, daß die Verbindung der Formel (II) ausgewählt ist unter 4'-Sulfo-3-benzylidencampher, 3-Benzylidencampher-10-sulfonsäure, 4'-Methyl-3'-sulfo-3-benzylidencampher, 4'-Chlor-3'-sulfo-3-benzylidencampher, 4'-Methyl-3-benzylidencampher-10-sulfonsäure, 3-(3-*tert.*-butyl-2-hydroxy-5-methyl)benzylidencampher-10-sulfonsäure, 3-(3-*tert.*-butyl-2-hydroxy-5-methoxy)benzylidencampher-10-sulfonsäure, 3-(3,5-di-*tert.*-butyl-4-hydroxy)benzylidencampher-10-sulfonsäure, 4'-Methoxy-3'-sulfo-3-benzylidencampher, 3-(4,5-Methylendioxy) benzylidencampher-10-sulfonsäure, 3-(4-Methoxy)benzylidencampher-10-sulfonsäue, 3-(4,5-Dimethoxy)benzylidencampher-10-sulfonsäure, 3-(4-n-Butoxy)benzylidencampher-10-sulfonsäure oder 3-(4-n-Butoxy-5-methoxy)benzylidencampher-10-sulfonsäure.

**17.** Verwendung nach Anspruch 12, dadurch gekennzeichnet, daß die Verbindung der Formel (b) folgende Formel aufweist:

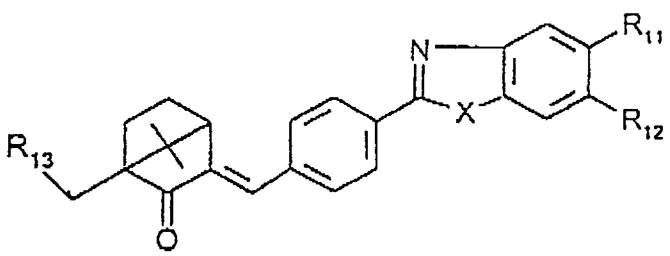

(III),

worin bedeuten:

- $R_{11}$ Wasserstoff, eine geradkettige oder verzweigte Alkyl- oder Alkoxygruppe mit etwa 1 bis 6 Kohlenstoffatomen oder eine Gruppe $-SO_3H$,

- $R_{12}$ Wasserstoff oder eine geradkettige oder verzweigte Alkyl- oder Alkoxygruppe mit etwa 1 bis 6 Kohlenstoffatomen,

- $R_{13}$ Wasserstoff oder eine Gruppe $-SO_3H$,

- wobei mindestens eine der Gruppen $R_{11}$ und $R_{13}$ eine Gruppe $-SO_3H$ bedeutet,

- X ein Sauerstoffatom oder Schwefelatom oder eine Gruppe -NR-, wobei R Wasserstoff oder eine geradkettige oder verzweigte Alkylgruppe mit etwa 1 bis 6 Kohlenstoffatomen bedeutet.

**18.** Verwendung nach Anspruch 17, dadurch gekennzeichnet, daß die Verbindung der Formel (III) die 2-[4-(Camphermethyliden)phenyl]benzimidazol-5-sulfonsäure ist.

**19.** Verwendung nach Anspruch 5, dadurch gekennzeichnet, daß die Verbindung die folgende Formel aufweist:

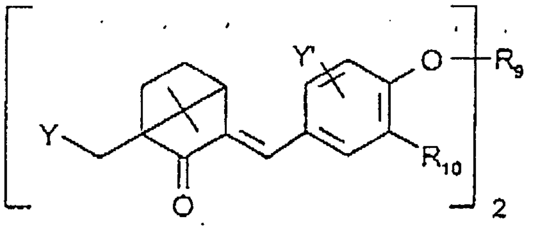

(c) ,

worin bedeuten:

- $R_9$ eine zweiwertige Gruppe $-(CH_2)_m-$ oder $-CH_2-CHOH-CH_2-$, wobei m eine ganze Zahl von 1 bis 10 ist ($1 \leq m \leq 10$),
- $R_{10}$ Wasserstoff oder eine Alkoxygruppe mit etwa 1 bis 4 Kohlenstoffatomen,
- Y und Y' Wasserstoff oder eine Gruppe $-SO_3H$, wobei mindestens eine der Gruppen Y oder Y' von Wasserstoff verschieden ist.

**20.** Verwendung nach Anspruch 19, daß die Verbindung der Formel (c) die Ethylen-bis-[3-(4'-oxybenzyliden)campher-10-sulfonsäure] ist.

**21.** Verwendung nach Anspruch 5, dadurch gekennzeichnet, daß die Verbindung folgende Formel aufweist:

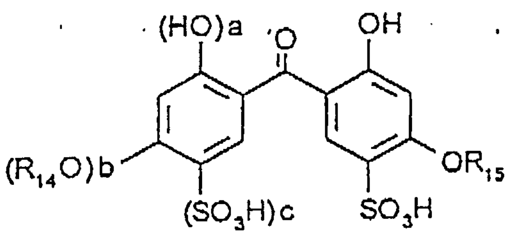

(d) ,

worin bedeuten:

- $R_{14}$ und $R_{15}$, die identisch oder voneinander verschieden sind, Wasserstoff oder eine geradkettige oder verzweigte Alkylgruppe mit etwa 1 bis 8 Kohlenstoffatomen,
- a, b und c, die identisch oder voneinander verschieden sind, Null oder 1.

**22.** Verwendung nach Anspruch 21, dadurch gekennzeichnet, daß die Verbindung der Formel (d) die 2-Hydroxy-4-methoxybenzophenon-5-sulfonsäure ist.

**23.** Verwendung nach Anspruch 5, dadurch gekennzeichnet, daß die Verbindung folgende allgemeine Formel aufweist:

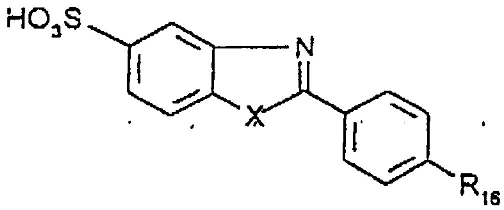

(e),

worin bedeuten:

- X Sauerstoff oder eine Gruppe -NH-,
- $R_{16}$ Wasserstoff, eine geradkettige oder verzweigte Alkyl- oder Alkoxygruppe mit etwa 1 bis 8 Kohlenstoffatomen oder eine Gruppe der Formel:

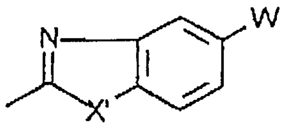

(IV),

worin X' Sauerstoff oder eine Gruppe -NH- und W H oder $SO_3H$ bedeutet.

**24.** Verwendung nach Anspruch 23, dadurch gekennzeichnet, daß die Verbindung der Formel (e) die 2-Phenylbenzimidazol-5-sulfonsäure, Benzol-1,4-di(benzimidazol-2-yl-5-sulfonsäure) oder Benzol-1,4-di(benzoxazol-2-yl-5-sulfonsäure) ist.

**25.** Verwendung nach einem der vorhergehenden Ansprüche, dadurch gekennzeichnet, daß die Verbindung in einem Mengenanteil von 0,01 bis 15 Gew.-% und noch bevorzugter von 0,5 bis 10 Gew.-% verwendet wird.

**26.** Verfahren zur kosmetischen Behandlung der Akne, das darin besteht, auf die Haut eine Verbindung aufzutragen, die mindestens eine Sulfonsäuregruppe aufweist, wobei die Sulfonsäuregruppen nicht vollständig neutralisiert sind.